# EUROPEAN PATENT APPLICATION

(11) **EP 2 037 277 A1**
(43) Date of publication of application: **18.03.2009**
(21) Application number: 07116148.3
(22) Date of filing: 11.09.2007
(51) Int. Cl.: G01N 33/86, G01N 33/49, G01N 35/10

(54) **Methods for assessing the functional integrity and the quality of a blood platelet containing sample**

(71) Applicant: F. Hoffmann-La Roche AG, 4070 Basel (CH); Roche Diagnostics GmbH, 68305 Mannheim (DE)
(72) Inventor: The designation of the inventor has not yet been filed
(74) Representative: Dick, Alexander

(57) **Abstract**

The present invention relates to a method for determining functional integrity of a blood platelet containing sample based on relating the baseline amount of a marker of platelet function to the amount of a marker of platelet function after bringing said marker into solution, and comparing the thus obtained value to a reference. Moreover, the invention relates to a method for quality control of a blood platelet containing sample. Further encompassed by the present invention are kits and devices for carrying out the methods of the present invention.

SEQUENCE LISTING

<110>  Roche Diagnostics GmbH
       F. Hoffmann La-Roche AG

<120>  Methods for assessing the functional integrity and the quality of
        a blood platelet containing sample

<130>  RD7784EP

<160>  1

<170>  PatentIn version 3.4

<210>  1
<211>  6
<212>  PRT
<213>  artificial

<220>
<223>  thrombin-receptor activation peptide

<400>  1

Ser Phe Leu Leu Arg Asn
1               5

## Description

The present invention relates to a method for determining functional integrity of a blood platelet containing sample based on relating the baseline amount of a marker of platelet function to the amount of a marker of platelet function after bringing said marker into solution, and comparing the thus obtained value to a reference. Moreover, the invention relates to a method for quality control of a blood platelet containing sample. Further encompassed by the present invention are kits and devices for carrying out the methods of the present invention.

Platelet substitution has become a major therapeutic tool for the treatment of, e.g, platelet disorders or thrombocytopenia. Worldwide approximately 20 million units of platelet concentrates are used for transfusion every year. Today, platelet concentrates to be used for platelet transfusion are mostly obtained by apheresis or from pools of buffy coats. Both methods require special equipment and trained personnel, and, therefore, are very cost-intensive. Generally, platelet concentrates are stored for up to five days after preparation. If the platelet concentrates are not used within these five days, the concentrates must be discarded due to quality aspects because the success of a platelet substitution significantly depends on the quality of the used platelet concentrates.

The process from the donation of a platelet concentrate to the transfusion of a platelet concentrate is a multi-step process (including donor selection, preparation, storage and distribution of the concentrate). Therefore, several aspects may affect the quality of a platelet concentrate, such as donor characteristics (smoking habits, inborn diseases, use of drugs inhibiting platelet function), bacterial contamination, the apheresis device, the storage buffer, the storage conditions, the storage time and the severity of platelet storage lesion.

At each major step from the selection of suitable donors to the administration, the quality of the concentrates is controlled. However, due to economic aspects or due to the lack of appropriate test methods, the measures that are undertaken as quality control only monitor the process step and do not assess the quality and the functionality of the platelet concentrate which is finally administered to a patient in need thereof.

The selection of suitable donors for platelet donation is a crucial step regarding the quality of platelet concentrates. Donors need to be selected whose platelets are functional and, therefore, can be successfully used for platelet transfusion. Donor characteristics that might affect the quality of a platelet concentrate are, e.g, the platelet count and the functionality of the platelets contained in the concentrate. However, only the platelet count is reliably determined, whereas usually no reliable tests are carried out in order to ensure a sufficient functionality of the donated platelet sample. However, it is well known that sufficient platelet functionality is important for the success of a platelet substitution. So far, platelet donors only have to to fill out a questionnaire before donating the blood sample. In the questionnaire the platelet donors have to answer questions, e.g., on previous intake of platelet function inhibiting drugs or on inborn diseases that might negatively affect platelet function. Generally, people with such diseases and/or previous intake of such drugs should be excluded from platelet donation. However, these questions are not always answered correctly, either knowingly or unknowingly. Thus, this measure does not allow for reliably reducing the risk of applying platelet concentrates with non functional platelets for platelet transfusion.

Another crucial step for the quality of a platelet concentrate is the preparation of the platelet concentrate itself. Nowadays, platelets concentrates are mostly obtained by apheresis (see above). However, it is generally assumed that the apheresis process itself impairs the platelet function of the collected platelets and, thus, negatively influences platelet function. Moreover, there is evidence that the degree of impairment caused by the apheresis process depends on the apheresis device used. So far, the routine testing that is done in order to check for damages to the platelets caused by apheresis is only based on visual inspection of the platelets samples. Discoloration or the absence of the so called swirling phenomenon which is based on light scattering by platelets of normal morphology during agitation indicates that a platelet concentrate is not suitable for platelet transfusion. Since the results of these visual tests are based on subjective interpretations, the may vary depending on the personnel carrying out these tests. Thus, it is difficult to standardize these visual tests and, therefore, they are known to be error-prone.

After apheresis, the concentrates are usually resuspended in synthetic buffers or plasma supplemented with specific additives. Both, the additives and the synthetic buffers shall limit the decrease of platelet function during storage. Generally, the concentrates are stored at 22°C +/- 2°C with gentle agitation. It is known that during platelet storage, the lactic acid level rises constantly resulting in a drop of the ph-value of stored platelet concentrates. As a consequence, the functionality of the platelet concentrates decreases. However, the measures undertaken for controlling platelet functionality during storage are indirect measures and, thus, measures that do not reliably determine platelet function. These measures encompass determining the ph-value of the concentrate and a visual inspection of the concentrates ("swirling phenomenon", see above). Moreover the storage temperature and the shaker speed are monitored during storage.

In order to provide patients that are in need of a platelet transfusion with a platelet concentrate, the concentrate needs to be transported. During the transportation step, platelet function might also decrease. However, platelet function is not determined after transporting platelet concentrates, only transport temperature and duration are recorded.

Before using a platelet concentrate for platelet substitution, there is also no direct and reliable determination of the functional integrity of platelets contained in the concentrate. Only after transfusion, the platelet increment is determined and occurring adverse reactions are recorded.

In general, methods for determining platelet function are known as such. Known methods for determining platelet function include optical aggregometry and impedance aggregometry (see e.g. the special textbook by Gawaz, M.: Blood platelets: physiology, pathophysiology, membrane receptors, antiplatelet principles, and therapy for atherothrombotic diseases. - Stuttgart; New York: Thieme, 2001. Pages 44-48).

In optical aggregometry, a sample of platelet-rich citrated plasma or a suspension of washed platelets is inserted into a light transmission photometer and stirred. After the addition of a platelet activator, the level of light transmission is monitored over time. Upon activation, the platelets start to form larger aggregates. This reduces the number of particles in the sample and results in an increase of light transmission. The faster the aggregates form, the higher is the aggregability of the platelets, i.e. the ability to form blood clots.

Impedance aggregometry is also based on the formation of aggregates from activated platelets. Instead of measuring the optical density of the sample, the electrical impedance of the sample is measured. In contrast to optical aggregometry, whole blood can be used for impedance aggregometry.

WO 99/14595 (Accumetrics Inc.) discloses a device for carrying out an aggregometric analysis of blood platelets.

However, all of these tests require a considerable amount of individual handling, which does not allow for high-throughput analysis. Specially trained personnel and special analyzers are required to carry out the methods, in the case of some methods restricting analysis to well-equipped clinics with special laboratories for blood analysis. In addition, these tests are very cost-intensive. Therefore, in order to avoid these costs, platelet concentrates are not routinely analyzed with respect to their functionality, and, thus, platelet concentrates with a low proportion of functional platelets may be used for platelet transfusion. One of the few measures undertaken to avoid the use of such concentrates is discarding platelet concentrates after five days of storage as precautionary measure. However, some of the concentrates may still be suitable for platelet transfusion.

Possible markers of platelet function of platelet concentrates (such as expression of CD62 on the platelet surface; see AuBuchon et al.; 2005 Transfusion 45:1356-1361 or expression of phosphatidylserines, the release of bioactive platelet-derived, inflammatory substances (e. g. TGF-β, PF4, RANTES or PDGF-AA, see Cognasse et al. Transfusion 46:1184-1189), metabolic parameters (e. g. pH, pCO₂, pO₂, LDH) have been reported as well as parameters morphological responses (e. g. platelet shape change, hypotonic shock response). The abundance of parameters proposed to assess the quality of platelet concentrates reflects the lack of a suitable marker of the functional integrity. None of the mentioned parameters has turned out as one simple assay for the determination of platelet quality under in vitro storage conditions.

Therefore, there is a need for means and measures which allow for a quick and reliable determination of the functional integrity of blood platelet containing samples. Moreover, there is a need for a quick and reliable method for quality control and for identifying platelet concentrates which are not functional.

The technical problem underlying the present invention can be seen as the provision of means and methods for complying with the aforementioned needs. The technical problem is solved by the embodiments characterized in the claims and herein below.

Accordingly, the invention relates to a method for determining the functional integrity of a blood platelet containing sample, comprising
a) determining in a first aliquot of said blood platelet containing sample the baseline amount of a marker of platelet function, or a variant of such marker, and
b) bringing into solution in a second aliquot of said blood platelet containing sample a marker of platelet function derived from the platelets, or a variant of such marker, and determining the amount of the marker of platelet function in the solution of said second aliquot, and
c) determining the functional integrity of said blood platelet containing sample by relating the baseline amount of a marker of platelet function as determined in a) to the amount of a marker of platelet function in solution as determined in b) and comparing the thus obtained value to a reference.

The method of the present invention, preferably, is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the determination in step (a) or (b), or a computer-implemented comparison in step (c). The present invention, also preferably, encompasses a method having the steps of the aforementioned method and the further step of obtaining a blood platelet containing sample from a subject.

Determining the functional integrity of a blood platelet containing sample as used herein means assessing, monitoring, confirming the functional integrity of a blood platelet containing sample. As will be understood by those skilled in the art, such a determination is usually not intended to be correct for all (i.e. 100%) of the examined blood platelet containing samples. The term, however, requires that the functional integrity of a blood platelet containing sample of a statistically significant portion of blood platelet containing samples can be assessed correctly. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, the functional integrity in at least 60%, at least 70%, at least 80% or at least 90% of blood platelet containing samples can be properly assessed by the method of the present invention.

The term "functional integrity of a blood platelet containing sample" (herein also referred to as "functional integrity of platelets in/of a blood platelet containing sample"), as used herein encompasses both the morphological integrity and the functionality of a blood platelet containing sample and, thus, relates to the capability of a platelet population that is contained in a said sample to form platelet aggregates, preferably, upon stimulation, i.e. the functional integrity indicates the capability of a platelet population to exert is physiological function. Therefore, the functional integrity, preferably, indicates whether a blood platelet containing sample is suitable for platelet transfusion. It is known in the art that the success of a platelet transfusion mainly relates on the capability of the transfused platelets to form aggregates.

Methods for obtaining blood platelet-containing samples are known by the person skilled in the art. The sample may be obtained by any means deemed appropriate, e.g. by drawing a blood sample from a vein or artery obtained from a subject.

The term "subject" as used herein relates to animals, preferably mammals, and, more preferably, humans. The sample may be drawn by means of a needle or a lancet. The blood platelet containing sample may be any kind of sample containing blood platelets. Said sample may be a sample of whole blood (e.g. venous blood or capillary blood) obtained from a subject. Moreover, the sample may be a pooled sample, thus a combination of samples that were obtained from more than one subject. Preferably, the sample is derived from a blood sample which has been further processed. More preferably, the blood platelet containing sample is a platelet concentrate. The term "platelet concentrate" is known as such. Platelet concentrates can be obtained and prepared by various methods which are known in the art. Platelet concentrates may be, preferably, prepared by various apheresis techniques (continuous or discontinuous). Furthermore, platelet concentrates may be, preferably, prepared by pooling from buffy coats, preferably from buffy coats from ABO identical donors. Platelet concentrates may also be obtained by separation from a whole blood sample. They may be stored in blood plasma or in optimized storage media containing additive solutions which are known in the art. Moreover, they may have been further processed, e.g. by leucodepletion.

Most preferably, a blood platelet containing sample in the context of the present invention is a platelet concentrate prepared by apheresis or pools from buffy coats.

Preferably, the blood platelet containing sample is to be used for blood transfusion and platelet transfusion, herein also referred to as platelet substitution. Platelet transfusions may be carried out to treat any kind of platelet disorders. Platelet disorders may be disorders which result in reduced number of platelets (thrombocytopenia) or an impaired platelet function. They may be also drug induced, e.g. by heparin or by chemotherapy. Other disorders or diseases encompass decreased platelet synthesis (bone marrow disease), loss of platelets (e.g., due to autoimmune disease or severe blood loss), high turnover of platelets in severe infectious diseases. The blood platelet containing sample may be stored after obtaining and/or preparing said sample, e.g. at least one day, two days, three days, four days, five days, six days, seven days, or ten days. Typically, platelet containing samples to be used for platelet substitution are stored for up to 5 days. Typically, the samples are stored at 22°C +/- 2°C with gentle agitation. They may be stored in different types of storage containers, e.g. in storage bags. In order to prevent coagulation, anticoagulants such as citrate may have been added to the sample. Moreover, the blood platelet containing sample may be stored under conditions which differ from the aforementioned conditions.

The volume of an aliquot of said sample may be in any range deemed appropriate and preferably allows reliable measurement. For example, the amount of the sample may be in the range of 1 µl to 500 ml, 10 µl to 100 ml, 100 µl to 50 ml, 0.5 ml to 20 ml, and/or 1 ml to 15 ml. Preferably, the volumes of the aliquot as used in step a) and step b) in the methods of the present invention are the same.

The method comprises the step of bringing a marker of platelet function derived from the platelets into solution. Preferably, the marker is derived from the platelet surface. This step can be carried out by any means deemed appropriate by the person skilled in the art, preferably, by adding a protease capable of cleaving of a fragment of the marker exposed on the platelet surface or by activating the platelets in the sample.

Also contemplated by the present invention, is that the methods of present invention are carried out after apheresis, after obtaining the blood platelet containing sample from pools of buffy coats, after resuspending blood platelets in plasma or any other suitable buffer (e.g. a synthetic buffer), before, during or after storage of the blood platelet containing sample, before or after transporting a blood platelet containing sample and/or shortly before administration. Therefore, the methods of the present invention are also methods for monitoring the functional integrity of a blood platelet containing sample in the individual steps from donation to administration. The methods of the present invention will also allow the optimization of the mentioned steps (thus, from donation to administration) with respect the functionality of the blood platelet containing sample.

Markers of platelet function are known to the person skilled in the art. In the context of the invention, any agent, particularly any protein or peptide, which is differentially released from and/or expressed on the surface of the platelets stimulated in vivo compared to platelets which have not been stimulated in vivo can be understood as a suitable marker of platelet function. Particularly, the marker is a marker which is present in the granular vesicles, which are platelet organelles and which fuse with the platelet membrane as part of the so-called "basic platelet reaction" after stimulation in vivo of the platelets, and more strongly expressed on the platelet surface after stimulation in vivo.

Preferred markers of platelet function are CD40L, sCD40L, P-selectin (CD62), glycoprotein IV (GP-IV), glycoprotein V (GP-V), glycoprotein VI (GP-VI, also known as p62), CD63(granulophysin), β-thromboglobulin (β-TG), platelet factor 4 (PF-4). Preferably, the marker is a membrane-bound marker, which can be shed from the platelet surface e.g. by activation in vitro and/or by action of a protease. Such markers include CD40L, P-selectin (CD62), GP-IV, GP-V, GP-VI, GP-Ib (CD42b), GP-IX. The respective fragments which are physiologically shedded by activation of the platelets are designated sCD40L (soluble CD40L), soluble P-selectin, soluble GP-IV, soluble GP-V, soluble GP-VI, soluble GP-Ib (Glycocalicin), soluble GP-IX. In contrast, β-TG and PF-4 are soluble molecules contained in the granular vesicles, which are released from the platelets upon stimulation or activation. CD63 (granulophysin) is contained in lysosomal vesicles and is released from the platelets upon stimulation or activation. In summary, suitable markers include CD40L, sCD40L, P-selectin, soluble P-selectin, GP-IV, soluble GP-IV, GP-V, soluble GP-V, GP-VI, soluble GP-VI, GP-Ib (CD42b), soluble GP-Ib, GP-IX, soluble GP-IX, CD63, platelet factor-4 (PF-4), β-Thromboglobulin (β-TG), or any variants thereof (see also Gardiner et al., J Thromb Haemost, 2007).

In a most preferred embodiment of the methods of the present invention the marker of platelet function is CD40L or sCD40L. sCD40L (soluble CD40 ligand) is a 18 kDa soluble protein found in blood. sCD40L is released by proteolysis from CD40L (CD40 ligand, also known as CD154), a 33 kDa type II transmembrane protein, which belongs to the TNF superfamily of transmembrane proteins. The structure and the composition of the protein is well known in the art, see, e.g. Karpusas et al., Structure 1995, 3:1019-39 or Anand et al., Thromb Haemost., 90:377-84. It has been suggested that 95% of sCD40L detectable in blood originates from blood platelets (Andre, P., et al. (2002): CD40L stabilizes arterial thrombi by a B3 integrin-dependent mechanism. Nature Medicine, vol. 8, 247-52.

The term "variants" in this context relates to proteins or peptides having an amino acid sequence which differs in at least one amino acid or which has at least one addition or deletion with respect to the aforementioned amino acid sequence. In particular, such a variant may be an isoform or allele which shows amino acid exchanges compared to the amino acid sequence of the most prevalent isoform in the human population referred to above. Preferably, such a variant has a sequence similarity to the most prevalent isoform of the protein or peptide of at least 70%, at least 80%, preferably at least 85%, more preferably at least 90%, most preferably at least 95%. Substantially similar are also fragments or degradation products, e.g. proteolytic fragments or degradation products, which are still recognized by the diagnostic means or by ligands directed against the respective full-length protein or peptide. In this context, it is referred also to the description and definitions of suitable fragments given in the context of bringing the marker of platelet function into solution by means of proteolysis. The term "variants" is also meant to relate to splice variants.

The term "variant" also relates to a post-translationally modified protein or peptide such as glycosylated peptide. A "variant" is also a protein or peptide which has been modified after collection of the sample, for example by covalent or non-covalent attachment of a label, particularly a radioactive or fluorescent label, to the protein or peptide.

Examples of particular variants and methods for their measurement are known. For example, there are different variants of sCD40L (see e.g. Pietravalle, F. et al. (1996). Human Native Soluble CD40L is a Biologically Active Trimer, Processed Inside Microsomes. J Biol Chem, vol. 271, pp. 5965-7).

Other embodiments of the invention include the measuring of different markers of platelet function in combination, simultaneously, i. e. within the same aliquot at the same time, or non-simultaneously.

The markers of platelet function are, preferably, exposed on the platelet surface upon stimulation of the platelets in vivo. Fragments of such surface-exposed markers can be brought into solution, for example by means of proteolysis. The fragments can be measured in solution by any means known to the person skilled in the art. By measuring the level of a thus cleaved-off fragment in solution, the degree of surface exposure of the respective marker on the platelets can be concluded. Preferably, the fragments cleaved off are of a size measurable by a suitable means, e.g. an antibody. Therefore, the size and/or sequence of the fragment should be specific for the respective marker to be determined. Depending on the particular sequence, a fragment is preferably of a size equal or larger than 4, 6, 8, 10, 12, 15, 18, 21, 25, or 30 amino acids. For example, suitable epitopes recognized by antibodies are larger than 10 amino acids. Smaller fragments (such as 4 amino acids or 6 amino acids in length) can be measured e.g. by means of mass spectrometry. Even measuring the amount of smaller fragments, such as 4 or 6 amino acids in length, may allow to conclude about the amount of surface-exposed marker, if the particular sequence of such fragments is specific for the marker, i.e. there are no other fragments of the same size and sequence in the sample originating from different proteins or peptides, or if the concentration of such different proteins or peptides is negligible.

A suitable protease may be of any kind deemed suitable by the person skilled in the art. Preferably, the protease is site-specific, i.e. it cuts the marker at a known site. However, the protease can also be rather unspecific, as long as reliable measurement of the fragment is possible. An example for a suitable protease may be the protease which naturally cleaves CD40L from the surface of the platelets (see e.g. Otterdal, K., Pedersen, T.M., and Solum, N.O. (2004). Release of soluble CD40 ligand after platelet activation - Studies on the solubilisation phase. Thrombosis Research, vol. 114, pp. 167-177). Examples for suitable proteases are described in US 6,861,504 to Phillips et al. (granted March 1, 2005) in the context of inhibitors of proteases whose target is CD40. Also for other markers suitable proteases can be easily determined by the person skilled in the art also for other markers, for example by means of software listing proteases for a given sequence. Such software, e. g. "PeptideCutter" is available to the person skilled in the art, e.g. via the website www.expasy.org. Many proteases are commerically available and can be easily tested by methods known to the person skilled in the art whether they are suitable for cleaving the respective surface-exposed marker of interest.

Site-specific proteases are known, for example Furin (available from New England Biolabs, Beverly, MA, USA) cuts preferably the sequence Arg-X-X-Arg, but with preference for the site Arg-X-X (Lys/Arg)-Arg.

The enzymatic action of the protease may be stopped by a suitable means, for example furine is inhibited by EGTA, α1-antitrypsine and by polyarginine compounds.

Suitable site-specific proteases can furthermore be identified by assays known to the person skilled in the art (see e.g. Grueninger-Leitch, F., Berndt, P., Langen, H., Nelbock, P. et al. (2000). Identification of β-secretase-like activity using a mass spectrometry-based assay system. Nature Biotechnology, vol. 18, pp. 66-70). The cited method is a high-throughput assay system for detecting site-specific protease from crude cell homogenates. The protein substrate is coupled to ceramic beads and incubated with cell homogenates; then the peptides are washed, released, and analyzed by mass spectrometry, allowing for purification of proteases that clip a protein at the site of interest.

The term "activating platelets" is known to the person skilled in the art. E.g. in the aggregometric methods known in the art, platelets are activated by contacting a sample with an activator of platelets. Preferably, platelet activation may be understood as relating to any method which results in shedding of sCD40L from the CD40L present on the surface of platelets, more particularly any method which results in shedding of a detectable amount, preferably of at least 30%, at least 50%, at least 70%, at least 80%, at least 90%, or at least 95% of the amount of CD40L present on the platelet surface within less than 10, 8, 7, 5, 3 or 2 minutes. The higher the fraction of surface exposed CD40L which is proteolyzed and liberates sCD40L, and/or the faster the shedding takes place, the higher the level of activation to be achieved will be. Alternatively or additionally, platelet activation may be understood as any method which results in shape changes typical of platelet activation (i.e. actin polymerisation and/or formation of pseudopodia, within less than 20, 10, 8, 7, 5, 3 or 2 minutes. The more pronounced the shape change is and/or the higher the fraction of platelets in the sample which show the shape change and/or the faster the shape change takes place, the higher the level of activation to be achieved will be. Again alternatively or additionally, platelet activation may be understood as any method which results in degranulation of dense granula and/or other granula and/or lysosomes from the platelets who less than 20, 10, 8, 7, 5 or 3 minutes. The more pronounced the degranulation is and/or the higher the fraction of platelets in the sample which show the degranulation and/or the faster the degranulation takes place, the higher is the level of activation achieved.

Preferably, activation is carried out exogenously, i.e. not by an activator already naturally present in the blood sample (such natural activators may include Collagen, Fibrinogen, Ca²⁺, von Willebrand Factor (vWF), Thrombin, serotonin in their naturally in the sample occurring concentrations). For example, a platelet activator may be added to the sample in order to carry out exogenous activation. Of course such exogenously added activator may have the same chemical structure as an activator already present in the sample (e.g. activators such as ADP may also be present as natural activators in the sample). However, activation by a naturally present activator may be considered if this process appears to be sufficiently controllable or standardizable.

The step of activating the platelets can be carried out by any means deemed appropriate, for example mechanically (e.g. by stirring the sample or by passing the sample through a fleece), electrically (e.g. by applying voltage to the sample), thermically (e.g. by applying heat to the sample), or chemically (e.g. by a platelet activator). The methods may also be combined, e.g. the fibers of a fleece can be coated with a platelet activator. Means for activating platelets, particularly platelet activators, can be found by their ability of induce platelet activation as described elsewhere in this specification. Platelet activation can be measured quite easily according to methods known in the art, see elsewhere in this specifcation. For example, a platelet activator can be found by contacting a blood sample with a candidate for a platelet activator and measuring the time until activation occurs, e.g. a shape change, or by measuring the extent of shedding of sCD40L from the surface of platelets. If the candidate reduces the time until activation in such a test compared to activation in a control sample not contacted with the candidate, then the candidate is identified as being a platelet activator. Similarly, if the candidate increases the amount of sCD40L shedded from the platelet surface or if such candidate reduces the time required for such shedding, then the candidate is identified as being a platelet activator.

Preferably, the platelet activator is not an activator of the plasmatic coagulation system ("a coagulation activator"). Such coagulation activators are occasionally added to blood samples in order to enhance blood coagulation. By releasing endogenous platelet activating agents from the sample, such coagulation activators may indirectly lead to an activation of blood platelets.

Examples for suitable platelet activators are known and include e.g. collagen, ADP, epinephrin, arachidonic acid, ristocetin, FIIa (thrombin), TRAP (thrombin-receptor activation peptide as known in the art; the peptide sequence is SFLLRN (SEQ ID NO:1), thromboxane A₂, PAF (platelet-activating factor), GPRP (the peptide gly-pro-arg-pro), serotonin, dopamine, collagen-related peptide, U-46619, and any synthetic analogues of said molecules, etc.. Preferably, a platelet activator to be used in the embodiments of the present invention is TRAP.

Activation is preferably carried out in a standardizable and/or controlled manner, e.g. by adding a platelet activator in a predetermined concentration, to the sample, for example 1-100, preferably 5-20 µM ADP, or 0.5-20, preferably 1-5 µg/ml collagen, or 1-100 µM TRAP, preferably 1-20 µM, more preferably 5µM TRAP.

The amount of a marker in solution in step b) of the methods of the present invention relates to the amount of the marker of platelet function that is in solution (solubilized) after bringing the marker of platelet function into solution. It is to be understood that this amount may not only encompasses the amount that has been brought into solution in step b) but additionally the amount of a marker of platelet function that already was present in solution prior to the solubilization step. In order to allow the determination of a marker of platelet function in solution, the resulting sample, preferably, is centrifuged after the solubilization step in order to obtain a supernatant containing the solved marker of platelet function. The supernatant may be separated from the pellet containing the platelets and possibly other blood cells.

The baseline amount of a marker of platelet function in step a) of the methods of the present invention, preferably, relates to the amount of a marker of platelet function that is present in a first aliquot of the blood platelet containing sample. Preferably, the baseline amount relates to the amount of a marker of platelet function in solution and not to a marker linked to the platelet surface or a marker inside intact platelets. Preferably, the marker has not been brought into solution by means and methods as described in this application (thus by means and methods of step b) of the methods of the present invention, e.g. by adding an activator). As it will be understood by the skilled person, the baseline amount of a marker of platelet function may increase during storage of a blood platelet containing sample, e.g. due to endogenous activation, storage conditions and/or platelet leakage. For determining the baseline amount, the aliquot of the blood platelet containing sample may be centrifuged and the baseline amount can be determined in the supernatant.

The marker of platelet function, preferably, is a peptide or polypeptide. Determining the amount of a marker of platelet function as referred to in this specification relates to measuring the amount or concentration, preferably semi-quantitatively or quantitatively. Measuring can be done directly or indirectly. Direct measuring relates to measuring the amount or concentration of a marker of platelet function, thus of a peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (a) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. Suitable antibodies are available e. g. from R&D systems, Bender MedSystems and Roche Diagnostics. E. g. in the case of P-selectin: Available from Bender MedSystems, including Biotin- or FITC-labeled antibodies and available from QED Bioscience Inc. ("clone AK-6). In the case of CD40L: Available from Antigenix America Inc. (including FITC-labeled antibodies), BD Biosciences Pharmingen, Bender MedSystems and others. In summary, suitable polyclonal, monoclonal, labeled- or unlabeled antibodies are widely available and can be obtained from commercial sources. Moreover, antibodies can be easily generated by the person skilled in the art according to well-known methods. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

Relating the baseline amount of a marker of platelet function to the amount of a marker of platelet function in solution as determined in step b) of the methods of the present invention as understood herein can be done by any method deemed appropriate. Preferably, relating said amounts can be done by subtracting the baseline amount of a marker of platelet function from the amount in solution (thus: amountₛₒₗᵤₜᵢₒₙ minus amount_{baseline}) or vice versa, or by determining the ratio of the amount of a marker in solution (that has been brought in solution) to the baseline amount (thus: ratio amountₛₒₗᵤₜᵢₒₙ to amount_{baseline}) or vice versa. More preferably, relating the said amounts means determining the ratio of said amounts, most preferably determining the ratio of the amount in solution (i. e., the amount as determined in step b) of the methods of the present invention) to the baseline amount (i. e., the amount as determined in step a) of the methods of the present invention).

The term "comparing" as used herein encompasses comparing the value determined by relating the baseline amount of a marker of platelet function as determined in step a) of the methods of the present invention to the amount of a marker of platelet function in solution as determined in step b) of the methods of the present invention, and comparing the thus obtained value to a suitable reference source specified elsewhere in this description. It is to be understood that comparing as used herein refers to a comparison of values. The comparison referred to in step c) of the methods of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the baseline amount as determined in step a) of the methods of the present invention to the amount as determined in step b) of the methods of the present invention to a reference it is possible to determine the functional integrity of a blood platelet containing sample and/or to assess the quality of a blood platelet containing sample. Preferably, said sample is be used for platelet transfusion. Therefore, the reference is to be chosen so that either a difference or a similarity in the compared amounts allows to determine the functional integrity of a blood platelet containing sample, to assess the quality of a blood platelet containing sample, and, thereby, to identify blood platelet containing samples which can be used for blood transfusion or which can not be used for blood transfusion.

Accordingly, the term "reference" as used herein refers to a value which allows determining functional integrity and/or the quality of a blood platelet containing sample. Accordingly, the reference may be derived from carrying out steps a) to b) of the methods of the present invention and relating the baseline amount of a marker of platelet function as determined in step a) of the methods of the present invention to the amount of a marker of platelet function in solution as determined in step b) of the methods of the present invention for a blood platelet containing reference sample, which, e.g, is known to have i) an insufficient functional platelet integrity and a low quality, or which is known to have a ii) a sufficient functional integrity and a high quality. The reference sample may be a sample known to have been successfully used for platelet transfusion (thus, to have achieved the targeted goals of the transfusion), or known not to have been successfully used for transfusion. Moreover, a suitable reference sample may be selected by the skilled person after assessing platelet function of said sample by optical aggregometry and impedance aggregometry. (These methods, however, are cost-intensive and require a considerable amount of individual handling, see above.).

Moreover, the reference may define a threshold value. In case, the ratio of the amount as determined in step b) to the baseline amount as determined in step a) of the methods of the present invention is determined (thus: the reference ratio of amountₛₒₗᵤₜᵢₒₙ to amount_{baseline}), a value larger than the threshold shall be indicative for a blood platelet containing sample with a high quality and a sufficient functional integrity, whereas a value lower than the threshold value shall be an indicator for a low quality and an insufficient functional integrity. "Good quality" and "sufficient functional integrity" in this context, preferably, means that a blood platelet containing sample is suitable for platelet transfusion and that, preferably, a platelet transfusion will be successful. Thus, a value larger than the threshold shall be indicative for a blood platelet containing sample suitable for blood transfusion, e.g. even after prolonged storage, wherein a value lower than the threshold shall be indicative for a sample not suitable for blood transfusion. It is to be understood that some characteristics of the blood platelet containing sample other than the characteristics mentioned in the context of the methods of the present invention might have to be assessed before using the blood platelet containing sample. Particularly, there may be a need to rule out bacterial contamination before using the sample for transfusion.

The reference applicable for a blood platelet containing sample may vary depending on the means used for the determination of a marker of platelet function referred to herein. Means for determining suitable references are known to the persons skilled in the art, e. g. a reference can be determined from Receiver-Operator-Curves (ROC). A preferred reference serving as a threshold may be derived from the upper limit of normal (ULN), i.e. the upper limit of the value as determined for blood platelet containing sample known to have a suitable quality and a sufficient functional integrity. The ULN for a given number of blood platelet containing samples can be determined by various well known techniques.

Preferably, the reference is a ratio, more preferably the reference is a ratio of the amount of a marker of platelet function as determined in step b) of the present invention to the baseline amount of a marker of platelet function as determined in step a) of the methods of the present invention (thus amountₛₒₗᵤₜᵢₒₙ to amount_{baseline}).

A ratio of the amountₛₒₗᵤₜᵢₒₙ to amount_{baseline} for the determination of the functional integrity of a blood platelet containing sample defining a threshold as referred to in accordance with the methods of the present invention is between 1.0 and 1.2, and, more preferably, 1.1.

Preferably, a value lower than the said ratio is indicative for a blood platelet containing sample that is not suitable for blood transfusion.

Preferably, a value larger than the said ratio is indicative for a blood platelet containing sample that is suitable for blood transfusion.

Advantageously, it has been found in the studies underlying the present invention that determining in a first aliquot of a blood platelet containing sample the baseline amount of a marker of platelet function, or a variant of such marker, and bringing, in a second aliquot of a blood platelet containing sample, a marker of platelet function derived from the platelets, or a variant of such marker into solution and determining the amount of the marker of platelet function in the solution of said second aliquot, and relating the determined baseline amount of a marker of platelet function to the determined amount of a marker of platelet function in solution, and comparing the thus obtained value to a reference is required for determining the functional integrity of a blood platelet containing sample. The described method is more reliable than prior art methods and in addition allows for a fast and easy determination of the functional integrity of a blood platelet containing sample. There have been several approaches in the prior art to assess the functional integrity of a blood platelet containing sample to be used for platelet transfusion by simple determining, e.g. the absolute values of a biochemical marker, or by physical or visual measures. However, these approaches turned out not to be very reliable (e.g., because they are based on subjective interpretations) or very cost-intensive (e.g., aggregometry). The studies underlying the present invention showed that by determining the above-mentioned value and comparing said value to a reference, the functional integrity of a blood platelet containing sample can be assessed. Thus, instead of measuring the absolute amount of a marker of platelet function, relating the amount of a marker of platelet function that has been brought into solution, e.g., by activating the platelets, to the baseline amount of said marker proved to be a valuable indicator of the functional integrity of a blood platelet containing sample. The methods of the present invention, if applied, will allow a simple determination of the functional integrity of platelets in a blood platelet containing sample and will be, particularly, beneficial regarding the use of platelet concentrates for platelet transfusion. As laid out in this application and as it is known in the art, a sufficient functional integrity of platelets positively influences the treatment success of a platelet transfusion. Particularly, a ratio of the amountₛₒₗᵤₜᵢₒₙ to amount_{baseline} larger than 1.1 indicates that a blood platelet containing sample has a sufficient functional integrity, whereas a ratio amountₛₒₗᵤₜᵢₒₙ to amount_{baseline} lower than 1.1 indicates that a blood platelet containing sample shows insufficient functional integrity. By providing a method for a particularly fast, easy and reliable determination of the function integrity of platelets, the present invention may allow for increased storage periods of platelet concentrates. As mentioned above, platelet concentrates are typically discarded after five days of storage due to quality aspects. Thanks to the methods of the present invention it may be possible to identify blood platelet containing samples which are suitable for blood transfusion, even after prolonged storage. Thus, platelet concentrates may be stored for more than five days. This will save costs and prevent a shortage of platelet concentrates. Moreover, the methods of the present will allow the identification of blood platelet containing samples that show insufficient platelet integrity and, thus, may be not suitable for platelet substitution, even when the samples have been stored for less than five days. Moreover, as the methods of the present invention allow for assessing the quality of a blood platelet containing sample, the methods will be valuable tools as methods for the optimization of devices used for platelet apheresis as well as for the optimization of platelet storage buffers for platelet concentrates.

Taken together, the methods of the present invention, if applied, will allow to determine functional integrity of a blood platelet containing sample by assessing both platelet integrity (by the baseline concentration of a marker of platelet function) and platelet function (by the concentration of a marker of platelet function that has been activated).

The present invention further relates to a method for quality control for a platelet containing sample comprising carrying out the steps a) and b) as laid out in any of the aforementioned methods and c) assessing the quality of said blood platelet containing sample by relating the amount as determined in step a) to the amount as determined in step b) and comparing the thus obtained value to a reference.

Unexpectedly, it has been found that the value as specified herein is not only advantageous for determining the functional integrity of a blood platelet containing sample, but also for assessing the quality of a blood platelet containing sample. It should be understood that a high quality of a sample (thus, with value equal or larger than 1.1 than the reference, as specified herein), preferably, indicates, that a sample should be preferred for transfusion. Thus, that the sample contains functional platelets compensating for the lack of platelets and/or restores platelet function in a patient (at least temporally), whereas a sample with a low quality (thus, with a value larger lower than 1.1than the reference, as specified herein), preferably, indicates the possibility of an unsuccessful platelet transfusion (thus, e.g., the targeted goals of the transfusion are not achieved what might, however, depend on some other unrelated patient characteristics). In the context of the studies underlying the present invention it has been found that the quality of a blood platelet containing sample correlates with the functional integrity of platelets.

Thus, we showed that the value determined in the methods of the present invention is a valuable tool not only to determine the functional integrity of a blood platelet containing sample but also to assess the quality of a blood platelet containing sample.

Therefore, the present invention also relates to a method for identifying a blood platelet containing sample being suitable for blood transfusion. Based on the value as determined by the methods of the present invention, it can be assessed whether a blood platelet containing sample is suitable for blood transfusion or not. As will be understood by those skilled in the art, such a determination is usually not intended to be correct for all (i.e. 100%) of the examined blood platelet containing samples (see above).

In addition, it is to be understood that the baseline amount as determined in step a) of the method of the present invention can be used to assess the risk of adverse side effect of using a blood platelet containing sample to be used for blood transfusion. Adverse side effects in the context of the present invention occur in a subject after receiving a platelet transfusion. These adverse side effects are known by the skilled person. Adverse side effects may be fever, allergic reactions or TRALI (transfusion-related acute lung injury). It is known in the art that the baseline amount of a marker of platelet function, particularly of sCD40L, correlates to the risk of said adverse side effects. Therefore, by determining the baseline amount (as in step a) of the methods of the present invention) and by comparing said amount to a suitable reference amount, also the risk of adverse side effects can be assessed.

The present invention also relates to a device adopted for carrying out the methods of the present invention comprising:
a) means for bringing in a blood platelet containing sample a marker of platelet function in solution, and
b) means for determining the baseline amount of a marker of platelet function, and means for determining the amount of a marker of platelet function in solution (i.e. a marker brought into solution by the means of a) , and
c) means for relating the baseline amount of a marker of platelet function to the amount of a marker of platelet function in solution as determined by the means of b), and
d) means for comparing a value determined by the means of c) to a reference, thereby allowing to determine the functional integrity and/or to assess the quality of said blood platelet containing sample.

The term "device" as used herein relates to a system of means comprising at least the aforementioned means operatively linked to each other as to allow the prediction. Preferred means for bringing a marker of platelet function in solution, determining the amount of a marker of platelet function in the solution, determining the baseline amount of a marker of platelet function as well as means for carrying out the comparison are disclosed above in connection with the methods of the present invention. How to link the means in an operating manner will depend on the type of means included into the device. For example, where means for automatically determining the amount of the peptides are applied, the data obtained by said automatically operating means can be processed by, e.g., a computer program in order to obtain the desired results. Preferably, the means are comprised by a single device in such a case. Said device may accordingly include an analyzing unit for the measurement of the amount of the marker of platelet function which, preferably, is a peptide or polypeptide in an applied sample and a computer unit for processing the resulting data for the evaluation. The computer unit, preferably, comprises a database including the stored reference amounts or values thereof recited elsewhere in this specification as well as a computer-implemented algorithm for carrying out a comparison of the determined amounts for the polypeptides with the stored reference amounts of the database. Computer-implemented as used herein refers to a computer-readable program code tangibly included into the computer unit. Alternatively, where means such as test stripes are used for determining the amount of the peptides or polypeptides, the means for comparison may comprise control stripes or tables allocating the determined amount to a reference amount. The test stripes are, preferably, coupled to a ligand which specifically binds to the peptides or polypeptides referred to herein. The strip or device, preferably, comprises means for detection of the binding of said peptides or polypeptides to the said ligand. Preferred means for detection are disclosed in connection with embodiments relating to the method of the invention above. In such a case, the means are operatively linked in that the user of the system brings together the result of the determination of the amount and the diagnostic or prognostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the peptides, Plasmon surface resonance devices, NMR spectrometers, mass-spectrometers etc.) and/or evaluation units/devices referred to above in accordance with the method of the invention.

Moreover, the present invention pertains to a kit adopted for carrying out the methods of the present invention, said kit comprising instructions for carrying out the said method and
a) means for bringing in a blood platelet containing sample a marker of platelet function in solution, and
b) means for determining the baseline amount of a marker of platelet function, and means for determining the amount of a marker of platelet function in solution (i.e. brought into solution by the means of a) , and
c) means for relating the baseline amount of a marker of platelet function to the amount of a marker of platelet function in solution as determined by the means of b), and
d) means for comparing a value determined by the means of c) to a reference, thereby allowing to determine the functional integrity and/or to assess the quality of said blood platelet containing sample.

The term "kit" as used herein refers to a collection of the aforementioned means, preferably, provided separately or within a single container. The components of the kit may be comprised by separate vials (i.e. as a kit of separate parts) or provided in a single vial. Moreover, it is to be understood that the kit of the present invention is to be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention.

Finally, the invention relates to the use of a marker of platelet function, preferably sCD40L, and means for determining said marker for determining the functional integrity of a blood platelet containing sample and assess the quality of the sample. Preferably, said sample is to be used for platelet substitution.

All references cited in this specification are herewith incorporated by reference with respect to their entire disclosure content and the disclosure content specifically mentioned in this specification.

The figures show:

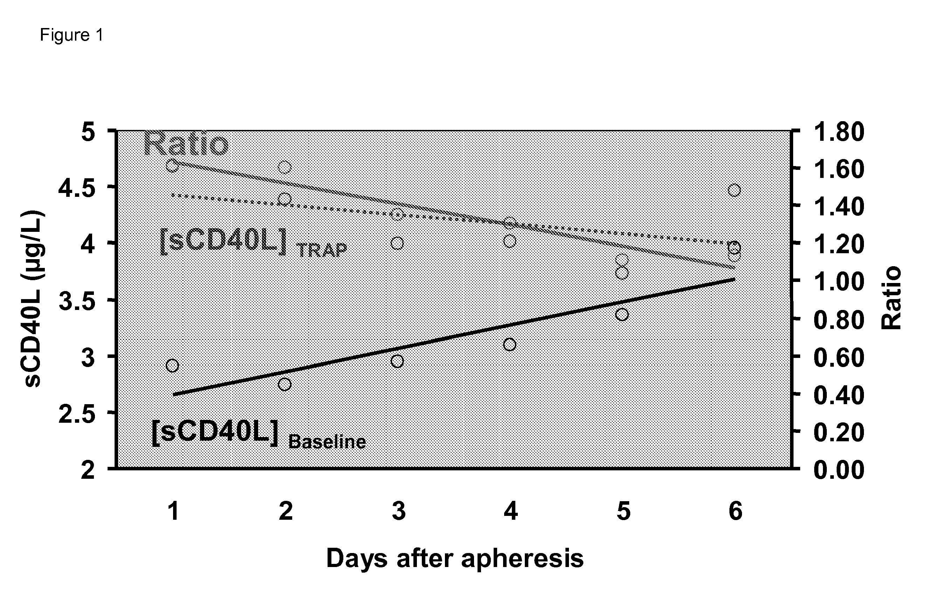

**Figure 1****:** Functional integrity of platelet concentrates after different storage periods.
   Platelet concentrates were prepared by apheresis and stored for up to six days. After different storage periods (indicated in days, x-axis), the baseline amount of sCD40L (sCD40L_{Baseline}) as well the amount of sCD40L after activation with TRAP (sCD40L_{TRAP} dotted line) were determined for individual platelet concentrates. In order to assess the functional integrity of a platelet concentrate, the ratio of the amount of sCD40L after activation (sCD40L_{TRAP}) to the baseline amount of sCD40L (sCD40L_{Baseline}) was determined (Ratio: sCD40L_{TRAP}/ sCD40L_{Baseline}).

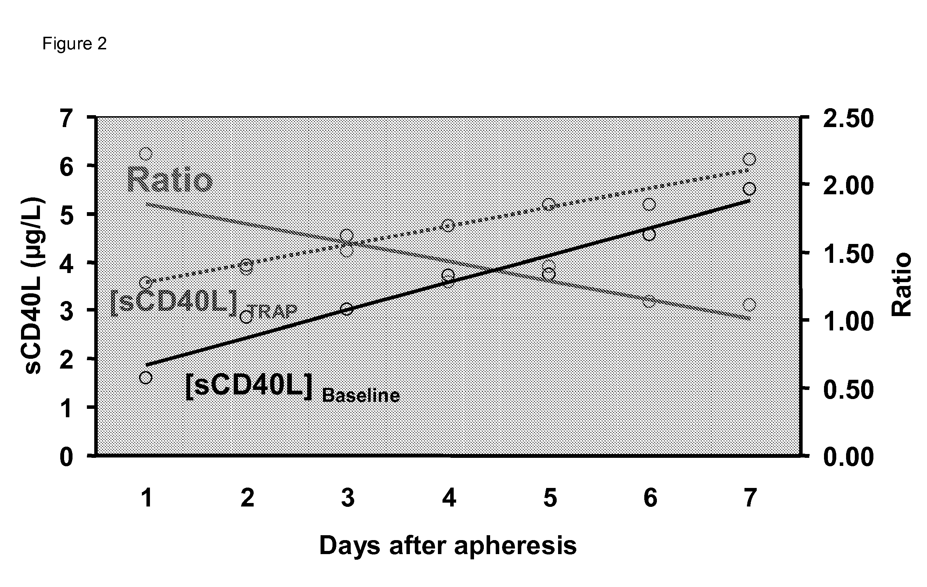

**Figure 2****:** Functional integrity of platelet concentrates after different storage periods.
   Platelet concentrates were prepared by apheresis and stored for up to seven days. After different storage periods (indicated in days, x-axis), the baseline amount of sCD40L (sCD40L_{Baseline}) as well the amount of sCD40L after activation with TRAP (sCD40L_{TRAP} dotted line) were determined for individual platelet concentrates. In order to assess the functional integrity of a platelet concentrate, the ratio of the amount of sCD40L after activation (sCD40L_{TRAP}) to the baseline amount of sCD40L (sCD40L_{Baseline}) was determined (Ratio: sCD40L_{TRAP}/ sCD40L_{Baseline}).

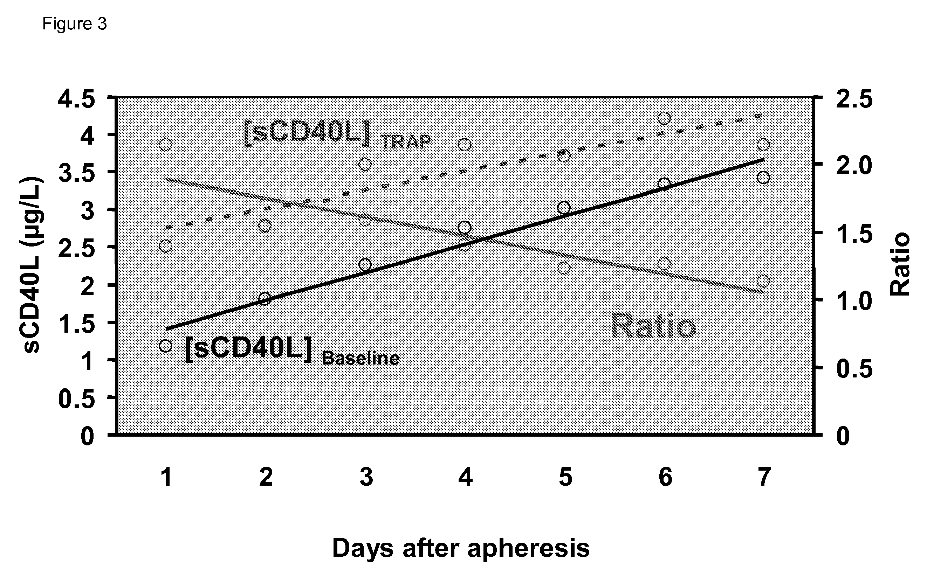

**Figure 3****:** Functional integrity of platelet concentrates after different storage periods.
   Platelet concentrates were prepared by apheresis and stored for up to seven days. After different storage periods (indicated in days, x-axis), the baseline amount of sCD40L (sCD40L_{Baseline}) as well the amount of sCD40L after activation with TRAP (sCD40L_{TRAP} dotted line) were determined for individual platelet concentrates. In order to assess the functional integrity of a platelet concentrate, the ratio of the amount of sCD40L after activation (sCD40L_{TRAP}) to the baseline amount of sCD40L (sCD40L_{Baseline}) was determined (Ratio: sCD40L_{TRAP}/ sCD40L_{Baseline}).

The following Example shall merely illustrate the invention. They shall not be construed, whatsoever, to limit the scope of the invention.

### Example: Determination of the functional integrity of a blood platelet containing sample.

Platelet concentrates were obtained by apheresis and stored for up to seven days under regular storage conditions known in the art. Functional integrity was determined as follows:
i) Aliquots (1 ml) of individual platelet concentrates that were stored for different time periods are centrifuged (10,000 x g, for 5 minutes at room temperature and the concentration of sCD40L in the supernatant is determined by use of an immunoassay for the Elecsys 2010 automated analyser (Roche Diagnostics GmbH, Germany).
ii) TRAP (SFLLRN (SEQ ID NO:1), thrombin receptor activating peptide) is added to a second aliquot (1 ml) of the respective platelet concentrates (final concentration of TRAP: 5 µM). After 10 minutes incubation, the second aliquot is centrifuged as described above and the amount of sCD40L in solution is measured as described above.

In order to assess the functional integrity of the analyzed sample, the ratio of the amount as determined in ii) to the amount as determined in i) is determined. Results are shown in Fig. 1, Fig. 2 and Fig. 3.

Thus, the methods of the present invention will allow, if applied, a reliable determination of the functional integrity of a blood platelet containing sample. This parameter allows for addressing the problems that can occur during the various steps from platelet donation to administration that may affect the quality of a platelet concentrate (as shown in table 1). Thus, the determination of one single parameter, the functional integrity of a platelet concentrate, can replace the conventional measures for quality control (see table 1).

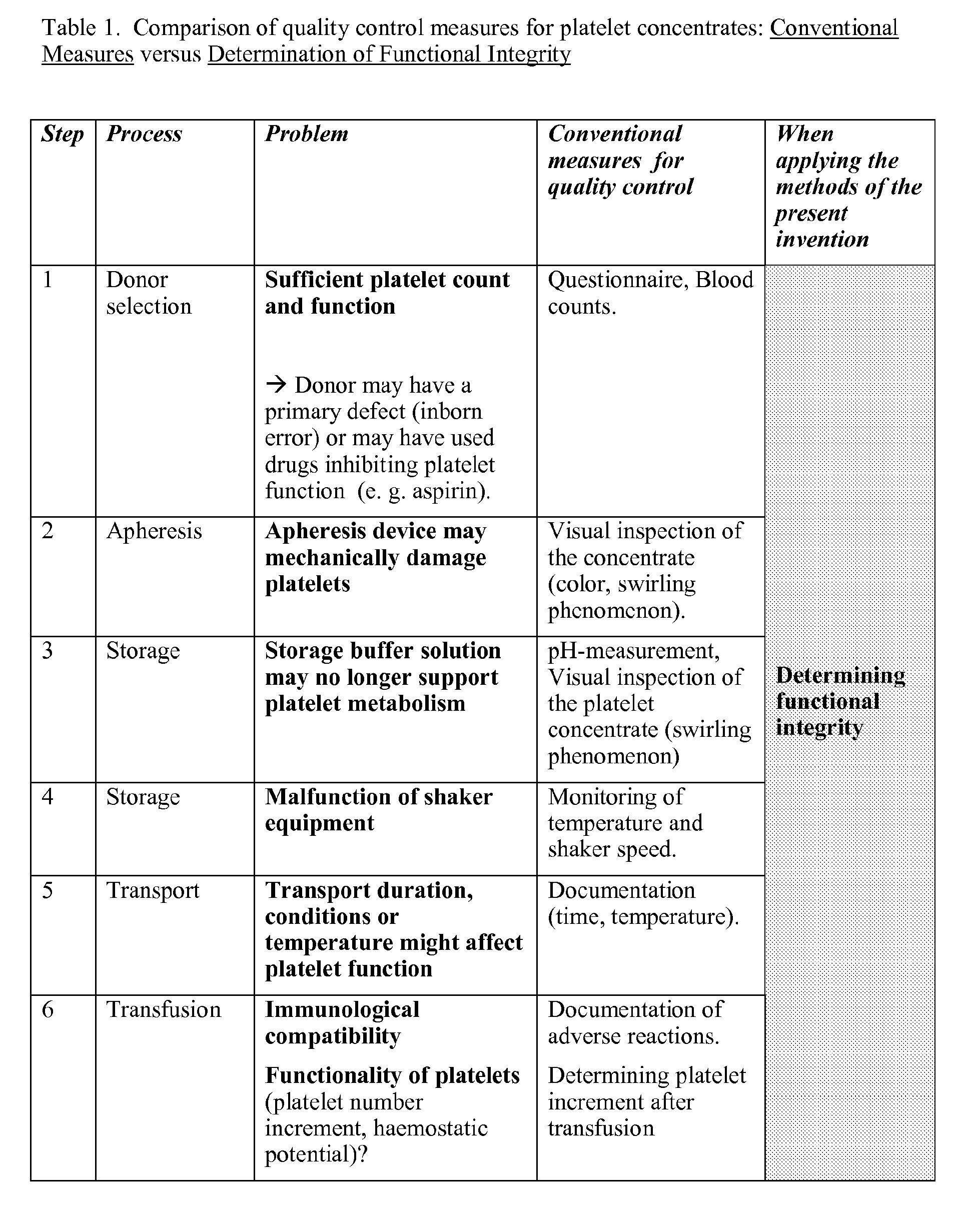

## Claims

1. A method for determining the functional integrity of a blood platelet containing sample comprising
a) determining in a first aliquot of said blood platelet containing sample the baseline amount of a marker of platelet function, or a variant of such marker, and
b) bringing into solution in a second aliquot of said blood platelet containing sample a marker of platelet function derived from the platelets, or a variant of such marker and determining the amount of the marker of platelet function in the solution of said second aliquot, and
c) determining the functional integrity of said blood platelet containing sample by relating the baseline amount of a marker of platelet function as determined in a) to the amount of a marker of platelet function in solution as determined in b) and comparing the thus obtained value to a reference.

2. The method of claim 1, wherein the marker of platelet function is CD40L or sCD40L.

3. The method of claims 1 to 2, wherein in step b) the sample is contacted with a platelet activator or a protease.

4. The method of any one of claims 1 to 3, wherein the blood platelet containing sample is a platelet concentrate.

5. The method of any one of claims 1 to 4, wherein in c) the relating is done by determining the ratio of the amount of a marker of platelet function in solution as determined in step b) to the baseline amount as determined in step a).

6. The method of claim 5 wherein the reference is a ratio of 1.1.

7. The method of any one of claims 5 to 6, wherein a ratio lower than the reference is indicative for a blood platelet containing sample that is not suitable for blood transfusion.

8. A method for quality control of a blood platelet containing sample comprising
a) determining in a first aliquot of said blood platelet containing sample the baseline amount of a marker of platelet function, or a variant of such marker, and
b) bringing into solution in a second aliquot of said blood platelet containing sample a marker of platelet function derived from the platelets, or a variant of such marker, and determining the amount of the marker of platelet function in the solution of said second aliquot, and
c) assessing the quality of said blood platelet containing sample by relating the baseline amount of a marker of platelet function as determined in a) to the amount of a marker of platelet function in solution as determined in b) and comparing the thus obtained value to a reference.

9. The method of claim 8, wherein the marker of platelet function is CD40L or sCD40L.

10. The method of claims 8 to 9, wherein in step (b) the sample is contacted with a platelet activator or a protease.

11. The method of any one of claims 8 to 10, wherein the blood platelet containing sample is a platelet concentrate.

12. The method of any one of claims 8 to 11, wherein in c) the relating is done by determining the ratio of the amount of a marker of platelet function in the solution as determined in step b) to the baseline amount as determined in step a).

13. The method of claim 12, wherein the reference is a ratio of 1.1.

14. The method of any one of claims 12 to 13, wherein a ratio lower than the reference is indicative for a blood platelet containing sample that is not suitable for blood transfusion.

15. A device for determining the functional integrity of a blood platelet containing sample adapted to carry out the method of any one of claims 1 to 7 comprising
a) means for bringing in a blood platelet containing sample a marker of platelet function in solution, and
b) means for determining the baseline amount of a marker of platelet function and the amount of a marker of platelet function in solution, and
c) means for relating the baseline amount of a marker of platelet function to the amount of a marker of platelet function in solution, and
d) means for comparing a value determined by the means of c) to a reference,
whereby the functional integrity of the blood platelet containing sample is determined.

16. A device for quality control of a blood platelet containing sample for blood transfusion adapted to carry out the method of any one of claims 8 to 14 comprising
a) means for bringing in a blood platelet containing sample a marker of platelet function in solution, and
b) means for determining the baseline amount of a marker of platelet function and the amount of a marker of platelet function in solution, and
c) means for relating the baseline amount of a marker of platelet function to the amount of a marker of platelet function in solution, and
d) means for comparing a value determined by the means of c) to a reference, whereby the quality of the blood platelet containing sample is assessed.

17. A kit for carrying out the method of any one of claims 1 to 7 comprising
a) means for bringing in a blood platelet containing sample a marker of platelet function in solution, and
b) means for determining the baseline amount of a marker of platelet function and the amount of a marker of platelet function in solution, and
c) means for relating the baseline amount of a marker of platelet function to the amount of a marker of platelet function in solution, and
d) means for comparing a value determined by the means of c) to a reference, whereby the functional integrity of the blood platelet containing sample is determined.

18. A kit for carrying out the method of any one of claims 8-14 comprising
a) means for bringing in a blood platelet containing sample a marker of platelet function in solution, and
b) means for determining the baseline amount of a marker of platelet function and the amount of a marker of platelet function in solution, and
c) means for relating the baseline amount of a marker of platelet function to the amount of a marker of platelet function in solution, and
d) means for comparing a value determined by the means of c) to a reference, whereby the quality of the blood platelet containing sample is assessed.
